# EUROPEAN PATENT SPECIFICATION

(11) **EP 3 222 239 B1**
(45) Date of publication and mention of the grant of the patent: **20.04.2022**
(21) Application number: 15861368.7
(22) Date of filing: 23.10.2015
(51) Int. Cl.: A61B 18/12

(54) **TREATMENT TOOL AND TREATMENT SYSTEM**
BEHANDLUNGSWERKZEUG UND BEHANDLUNGSSYSTEM
OUTIL DE TRAITEMENT ET SYSTÈME DE TRAITEMENT

(30) Priority: 18.11.2014 JP 2014233825
(43) Date of publication of application: 27.09.2017
(73) Proprietor: Olympus Corporation, Hachioji-shi, Tokyo 192-8507 (JP)
(72) Inventor: TAKASHINO, Tomoyuki, Hachioji-shi, Tokyo 192-8507 (JP); TAKEI, Yusuke, Hachioji-shi, Tokyo 192-8507 (JP); TANAKA, Kazuhiro, Hachioji-shi, Tokyo 192-8507 (JP); TANAKA, Kazue, Hachioji-shi, Tokyo 192-8507 (JP)
(74) Representative: Gunzelmann, Rainer
(86) International application number: PCT/JP2015/079961
(87) International publication number: WO 2016/080147

(56) References cited:
- JP-A- 2002 537 938
- JP-A- 2003 175 053
- JP-A- 2005 514 102
- JP-A- 2007 037 568
- JP-A- 2008 018 226
- US-A1- 2008 015 567
- US-A1- 2008 015 575

## Description

### Technical Field

The invention relates to a treatment instrument and a treatment system.

### Background Art

WO 2003/057058 A1 discloses a treatment instrument in which a stapler, a thermal cutting element, and cauterizing elements are disposed. The treatment instrument activates a cauterizing element to heat living tissue to a temperature sufficient to sever and seal (coagulate) the tissue, and then activates a stapler to dispense staples simultaneously with cauterizing. After the above procedure, a cutting element is activated to sever the stapled tissue.

The treatment by the treatment instrument disclosed in WO 2003/057058 A1 requires three steps of actions: sealing living tissue by using energy; dispensing staples; and severing the sealed tissue. Alternatively, the treatment requires two steps of actions: sealing living tissue by using energy and dispensing staples simultaneously, and thereafter severing the sealed tissue. In the treatment using the treatment instrument disclosed in WO 2003/057058 A1, the next step is performed after confirming the completion of the previous step. Thus, it takes a long time to complete all the treatments when the same treatment is repeated.

US 2008/0015567 A1 discloses a treatment device having a pair of first, second jaws capable of opening/closing with respect to each other at the tip end portion and comprises a tissue pressing portion in the relatively dull shape provided on a surface portion of the first jaw opposite to the second jaw and having a projection portion formed projecting toward the second jaw side, a receiving member provided on a surface portion of the second jaw opposite to the first jaw at a position opposite to the tissue pressing portion, and a plurality of electrode portions provided at least at one of the first jaw and the second jaw so that a high-frequency current flows through a living tissue compressed by the tissue pressing portion and the receiving member.

US 2008/0015575 A1 discloses an electrode assembly having a pair of opposing first and second jaw members. Each jaw member includes an electrically conductive tissue sealing surface extending along a length thereof, each tissue sealing surface is adapted to connect to a source of electrosurgical energy such that the tissue sealing surfaces are capable of conducting electrosurgical energy through tissue held therebetween to effect a seal. The assembly includes at least one heating element disposed within at least one of the jaw members. The heating element is configured to pre-heat the electrically conductive tissue sealing surfaces before electrosurgical energy is applied.

### Summary of Invention

The present invention is to provide a treatment instrument according to claim 1 and a treatment system according to claim 7 capable of incising living tissue by one action while maintaining a high sealing (coagulating) performance. Further aspects of the invention are disclosed in the dependent claims.

### Brief Description of Drawings

FIG. 1 is a schematic diagram of a treatment system according to the first to sixth embodiments.
FIG. 2A is a schematic side view of a treatment instrument of a treatment system according to the first embodiment in a state where a treatment portion of the treatment instrument is opened.
FIG. 2B is a schematic side view of the treatment instrument of the treatment system according to the first embodiment in a state where the treatment portion is closed.
FIG. 3A is a schematic top view of the treatment instrument of the treatment system according to the first embodiment when in a state where the treatment portion protrudes in a straight line to a tubular body.
FIG. 3B is a schematic diagram of a rotating mechanism within a handle in a state where the treatment portion of the treatment instrument of the treatment system according to the first embodiment protrudes in a straight line relative to the tubular body.
FIG. 4A is a schematic diagram of the treatment portion of the treatment instrument of the treatment system according to the first embodiment in a state where the treatment portion is rotated relative to the tubular body when viewed from above.
FIG. 4B is a schematic diagram of the rotating mechanism within the handle in a state where the treatment portion of the treatment instrument of the treatment system according to the first embodiment is rotated relative to the tubular body.
FIG. 5A is a schematic cross-sectional view of the treatment portion of the treatment instrument of the treatment system according to the first embodiment in a state where the treatment portion is opened.
FIG. 5B is a schematic cross-sectional view of the treatment portion of the treatment instrument of the treatment system according to the first embodiment, in a state where the treatment portion is closed, taken along line 5B-5B in FIGS. 2B and 3A.
FIG. 5C is a schematic cross-sectional view of the treatment portion of the treatment instrument of the treatment system according to the first embodiment, in a state where the treatment portion is closed, taken along line 5C-5C in FIGS. 2B and 3A.
FIG. 5D is a schematic cross-sectional view illustrating a modification of FIG. 5C, in a state where the treatment instrument of the treatment system according to the first embodiment is closed, taken along line 5C-5C in FIGS. 2B and 3A.
FIG. 6 shows schematic graphs representing a state where energy is sequentially output from a high frequency energy source and a heat energy source to the treatment instrument of the treatment system according to the first embodiment, with a horizontal axis representing time and a vertical axis representing an output power from the high frequency energy source, a temperature of a heating element, and a change in temperature of living tissue due to an output of energy.
FIG. 7 is a schematic flowchart representing a state where living tissue is sealed and incised by one action by using the treatment system according to the first embodiment.
FIG. 8A shows schematic graphs representing a state where energy is sequentially output from a high frequency energy source and a heat energy source to the treatment instrument of the treatment system according to the first embodiment, with a horizontal axis representing time and a vertical axis representing an output power from the high frequency energy source and the temperature of the heating element.
FIG. 8B shows schematic graphs representing a state where energy is sequentially output from the high frequency energy source and the heat energy source to the treatment instrument of the treatment system according to the first embodiment, with a horizontal axis representing time and a vertical axis representing an output power from the high frequency energy source and the temperature of the heating element.
FIG. 8C shows schematic graphs representing a state where energy is simultaneously output from the high frequency energy source and the heat energy source to the treatment instrument of the treatment system according to the first embodiment, with a horizontal axis representing time and a vertical axis representing an output power from the high frequency energy source and the temperature of the heating element.
FIG. 9 is a schematic cross-sectional view of the treatment portion of the treatment instrument of the treatment system according to the second embodiment in a state where the treatment portion is opened.
FIG. 10 is a schematic cross-sectional view of the treatment portion of the treatment instrument of the treatment system according to the third embodiment in a state where the treatment portion is opened.
FIG. 11 is a schematic cross-sectional view of the treatment portion of the treatment instrument of the treatment system according to the fourth embodiment in a state where the treatment portion is opened.
FIG. 12 is a schematic cross-sectional view of the treatment portion of the treatment instrument of the treatment system according to the fifth embodiment in a state where the treatment portion is opened.
FIG. 13 is a schematic cross-sectional view of the treatment portion of the treatment instrument of the treatment system according to the sixth embodiment in a state where the treatment portion is opened.

### Description of Embodiments

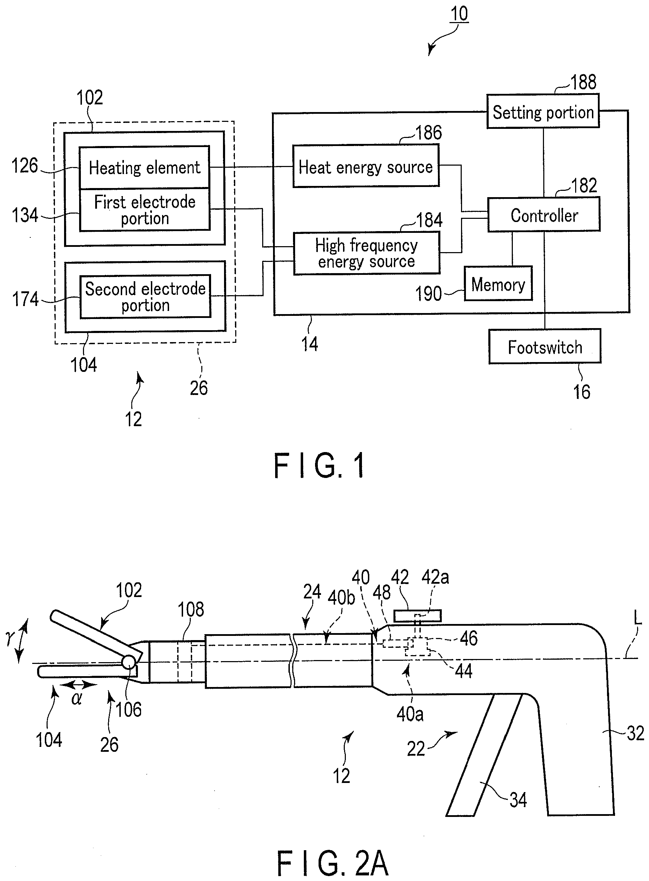

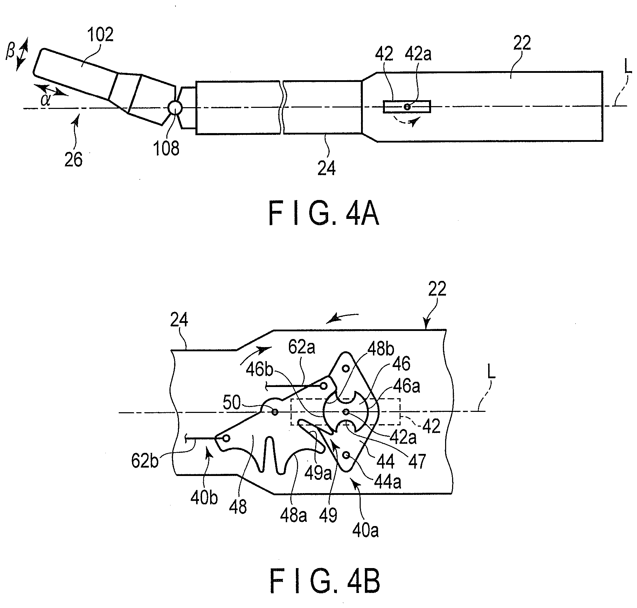

Hereinafter, embodiments for implementing the present invention will be explained with reference to FIGS. 1 to 13.

The first embodiment will be described with reference to FIG. 1 to FIG. 8C.

As shown in FIG. 1, the treatment system 10 according to the present embodiment includes a treatment instrument 12, and a control section 14. The control section 14 is connected to a footswitch 16 as an example, which is detachable and attachable relative to the control section 14. In the following example, the footswitch 16 that is operable by a foot is used; however, a handswitch which is provided in the treatment instrument 12 and is operable by a hand of an operator may be used instead.

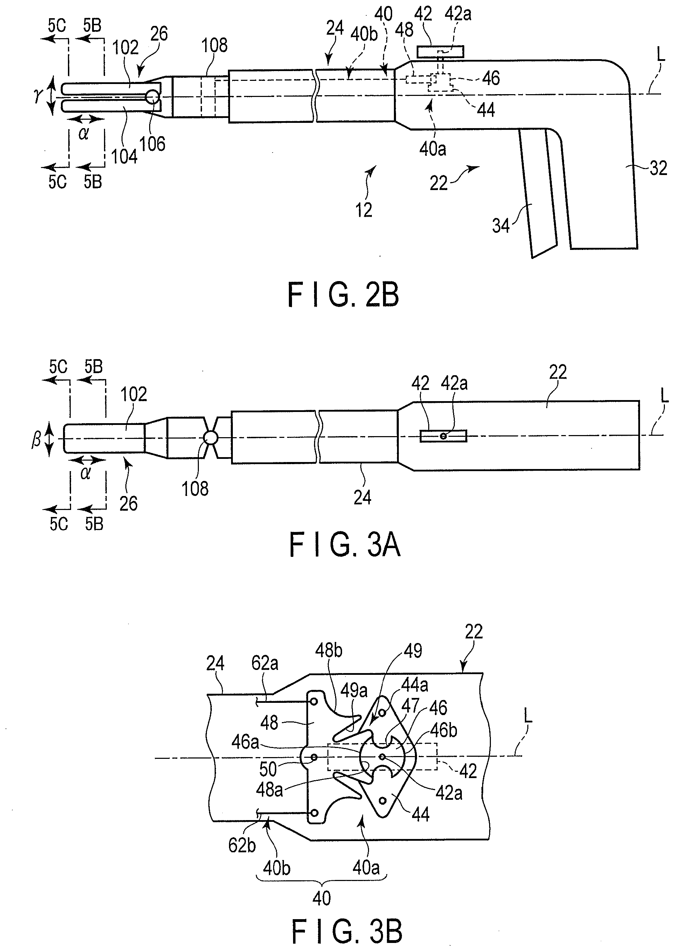

As shown in FIGS. 2A and 2B, the treatment instrument 12 includes an operation portion 22, a tubular body 24 disposed at a distal end of the operation portion 22, and a treatment portion 26 disposed at a distal end of the tubular body 24. The tubular body 24 is made of a material having rigidity, such as stainless steel, and inner and outer peripheral surfaces thereof are covered with a material having electrical insulation properties.

The operation portion 22 is formed of a plastic material having electrical insulation properties, for example. The operation portion 22 includes a stationary handle 32 and a movable handle 34. By the operation of the movable handle 34, a first jaw 112 and a second jaw 152 (described later) can be simultaneously moved toward a close direction or an open direction. Alternatively, by the operation of the movable handle 34, the first jaw 112 can be moved toward the close direction or the open direction, while the position of the second jaw 152 is fixed. That is, the first and second jaws 112 and 152 can be opened or closed relatively by selectively using an appropriate known mechanism. Accordingly, a distance between a first and second holding surfaces 132 and 172 opposed to each other in the treatment portion 26, which is described later, can increase or decrease by the operation of the movable handle 34 with the known mechanism. For simplification of explanation, it is assumed that the first jaw 112 moves relative to the second jaw 152 by the operation of the movable handle 34.

The treatment instrument 12 includes a rotating mechanism 40 which is configured to maintain a state where the treatment portion 26 is rotated by a predetermined angle relative to the distal end of the tubular body 24. The rotating mechanism 40 is preferably formed as a combination of a unidirectional power transmission mechanism 40a which is like a Geneva device and a parallelogram link mechanism 40b, for example, as shown in FIGS. 2A to 4B. With the preferred structure, the treatment portion 26 is rotated only when a user actively operates the rotating mechanism 40 to rotate a rotating lever 42, and the treatment portion 26 is prevented to be passively rotated when the rotating mechanism 40 is not operated.

A transmission mechanism 40a includes the rotating lever 42, an input side rotating portion 44, an input cam 46, and an output side rotating portion 48. The treatment portion 26 is connected to the output side rotating portion 48 of the transmission mechanism 40a by the known parallelogram link mechanism 40b, for example. Accordingly, if the rotating lever 42 is rotated around the axis of the rotation shaft 42a to exceed a predetermined angle, the treatment portion 26 can be rotated by a predetermined angle linked with the rotating shaft 108 placed at the distal end of the known parallelogram link mechanism 40b (described later).

It is preferable to place the rotating lever 42 on a top surface of the operation portion 22 to be operable by a user. The rotating lever 42 includes a rotation shaft 42a in a direction orthogonal to a central axis of the tubular body 24. It is preferred that the input side rotating portion 44, the input cam 46, and the output side rotating portion 48 are placed inside the operation portion 22.

A first projection 46a of the input cam 46 integrally formed with the input side rotating portion 44 is slidably engaged with a first recess 48a of the output side rotating portion 48. If the rotation force of the input cam 46 by the rotating lever 42 is equal to or less than a predetermined rotation amount (which is discretionarily set, 180° in this embodiment, but may be 90° or 120°, etc.), the rotation force is not transmitted to the output side rotating portion 48. On the other hand, if the output side rotating portion 48 is rotated due to addition of an external force to the treatment portion 26, the input cam 46 does not move, and the first recess 48a of the output side rotating portion 48 merely rotates within an appropriate range. Accordingly, the rotation force is not transmitted to the input cam 46 from the output side rotating portion 48.

If the rotation of the input cam 46 by the rotating lever 42 is greater than the predetermined rotation amount, an engagement groove 49a of the output side rotating portion 48 is thrust by a pin 44a of the input side rotating portion 44, and the output side rotating portion 48 is rotated around the axis of a rotation shaft 50. When the output side rotating portion 48 is rotated, one wire 62a of the parallelogram link mechanism 40b that is connected to the output side rotating portion 48 is pulled back, and the other wire 62b is moved forward. By means of the parallelogram link mechanism 40b, the treatment portion 26 rotates by a predetermined angle relative to the distal end of the tubular body 24. In this case, the first recess 47 of the input cam 46 bridges a first projection 49 of the output side rotating portion 48. Then, a second projection 46b of the input side cam 46 is slidably fit into a second recess 48b of the output side rotating portion 48.

The transmission mechanism 40a does not transmits the rotation force of the input cam 46 to the output side rotating portion 48 when the second projection 46b of the input cam 46 formed integrally with the input side rotating portion 44 is slidably fit into the second recess 48b of the output side rotating portion 48. On the other hand, if the output side rotating portion 48 is rotated due to the addition of an external force to the treatment portion 26, the rotation force is not transmitted to the input cam 46.

Accordingly, the transmission mechanism 40a transmits the rotation force from the input side rotating portion 44 to the output side rotating portion 48 by means of the rotating lever 42, but does not transmit the rotation force from the output side rotating portion 48 to the input side rotating portion 44. By using the rotating mechanism 40, the treatment portion 26 is prevented from being unintentionally rotated. If the rotating lever 42 is rotated to exceed the predetermined rotation angle, the treatment portion 26 can be rotated by a preset angle relative to the distal end of the tubular body 24.

As shown in FIGS. 2A and 2B, the treatment portion 26 includes a first treatment piece 102, a second treatment piece 104, and an opening-and-closing shaft 106 which opens and closes the first and second treatment pieces 102 and 104 relatively. As described above, the opening-and-closing shaft 106 is used to rotate the first jaw 112 of the first treatment piece 102 in this embodiment, but may be used to rotate the second jaw 152 of the second treatment piece 104. The rotating shaft 108 is disposed between the opening-and-closing shaft 106 and the distal end of the tubular body 24, namely, the proximal end side of the opening-and-closing shaft 106. The first and second treatment pieces 102 and 104 are respectively defined by a longitudinal direction α, a width direction β, and an open/close direction γ (thickness direction), each of which is orthogonal to each other. The first and second treatment pieces 102 and 104 are respectively formed so that the length of the longitudinal direction α is longer than that of the width direction β, and the open/close direction γ, and the length of the width direction β is longer than that of the open/close direction γ.

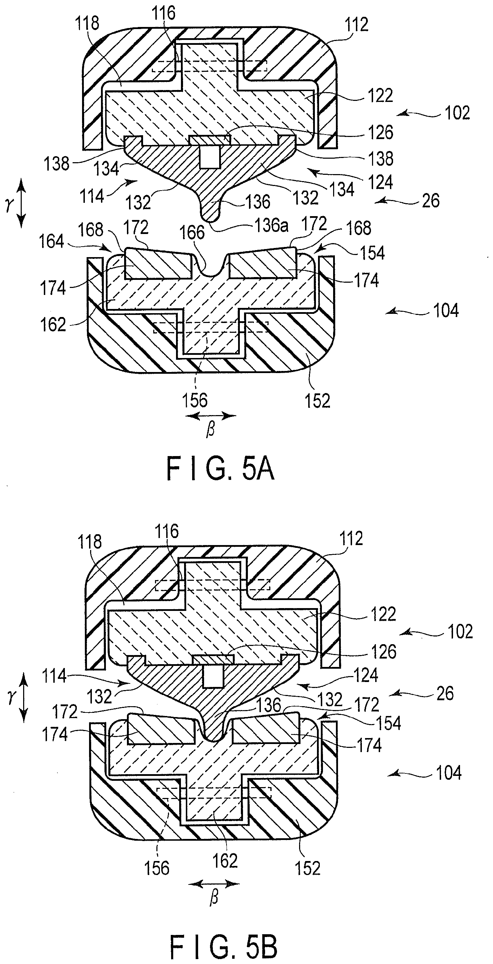

FIG. 5A illustrates a cross section of the treatment portion 26 shown in FIG. 2A, and FIG. 5B illustrates a cross section of the treatment portion 26 shown in FIG. 2B.

As shown in FIGS. 5A and 5B, the first treatment piece 102 includes the first jaw (upper-side jaw) 112 and a first holding body 114. The first jaw 112 preferably includes a material which has electrical insulation properties and heat resistance.

The first jaw 112 moves in the open/close direction γ by means of the opening-and-closing shaft 106 relative to the second treatment piece 104, in accordance with the operation of the movable handle 34. In this embodiment, a pin 116 is disposed between the first jaw 112 and the first holding body 114, in parallel to the width direction β. Accordingly, the first holding body 114 is shakable around the axis of the pin 116 like a seesaw, relative to the first jaw 112. This structure realizes uniformly adding a holding force to living tissue held between the first and second treatment pieces 102 and 104, in the longitudinal direction α.

A gap 118 is defined between the first jaw 112 and the first holding body 114. The gap 118 functions as a heat insulating layer, and prevents heat by a heating element 126 described below from being transmitted to the first jaw 112, to the greatest extent possible.

It may be preferable that a cover that forms the gap (air layer) 118 (not shown in the drawings) between the first holding body 114 and the first jaw 112 is placed to the first holding body 114. In addition, it may be preferable that the first jaw 112, the cover, and the first holding body 114 are swingably supported by the pin 116.

The first holding body 114 is closer to the second treatment piece 104, in comparison with the first jaw 112. The first holding body 114 includes a main body 122 swingably supported relative to the first jaw 112, a holding body 124 fixed to the main body 122, and a heating element (heater) 126 disposed between the main body 122 and the holding body 124. The heating element 126 is formed, for example, of a resistive thin film, etc. The heating element 126 heats a first holding surface 132 to a second temperature T2 which is higher than a first temperature T1 and is sufficient to incise living tissue held between the first and second holding surfaces 132 and 172. It is preferable that the holding body 124 and the heating element 126 are formed to be longer in the longitudinal direction α than in the width direction β.

The total length of the main body 122, the holding body 124, and the heating element 126 in the open/close direction (thickness direction) γ is formed to be shorter than in the longitudinal direction α, but may be longer, shorter, or equal, in relation to the width direction β.

The main body 122 is formed of a material which has electrical insulation properties and heat resistance. The main body 122 is preferably formed of a material which has low thermal conductivity. Accordingly, it is possible to prevent heat from moving toward the first jaw 112 to the greatest extent possible, when the heating element 126 generates heat. On the other hand, the holding body 124 is formed of a material which has good conductivity and good thermal conductivity, such as copper alloy material, and aluminum alloy material, for example.

The holding body 124 includes a pair of first holding surfaces 132, each of which includes a first electrode portion (first high frequency electrode portion) 134, and a blade 136 that is placed adjacent to the first holding surfaces 132. The blade 136 is consecutively and integrally formed between the first holding surfaces 132 and formed of the same material as the first holding surfaces 132. Accordingly, the blade 136 is used as part of the first electrode portion 134. A projected surface 136a (described later) between the pair of first holding surfaces 132 and the blade 136 is formed as a smoothly curved surface.

Each of the pair of first holding surfaces 132 is formed as an inclined surface which is inclined relative to a virtual surface defined by the longitudinal direction α and the width direction β of the holding body 124. The pair of first holding surfaces 132 each formed as an inclined surface are close to the second holding surfaces 172 (described later) of the second treatment piece 104 at the center of the width direction β. The second holding surfaces 172 are opposed to the first holding surfaces 132, and holds living tissue together with the first holding surfaces 132. The first holding surfaces 132 are preferably formed to become farther away from the second holding surfaces 172 toward a side surface 138 of the holding body 124 in the width direction β. Accordingly, the thickness (in the open/close direction γ) of the holding body 124 except the blade 136 is formed to increase toward the center in the width direction β, and decrease toward the side surface 138.

The blade 136 is preferably formed at the center in the width direction β of the holding body 124. The blade 136 has the projected surface (region for applying pressure for incision) 136a which applies greater pressure to living tissue in comparison with the pair of first holding surfaces (region for applying pressure for sealing) 132. The projected surface 136a is provided to the first holding surfaces 132 and projects from the first holding surfaces 132 to the second holding surfaces 172. The projected surface 136a is smoothly consecutive to both of the pair of first holding surfaces 132. The projected surface 136a incises living tissue held between the first and second holding surfaces 132 and 172, by applying pressure and heat transferred from the heating element to the living tissue.

The projected surface 136a projects in the longitudinal direction α. The projected surface 136a is preferably formed to have the length equal to or slightly shorter than the main body 122 in the longitudinal direction α. The distal end of the projected surface 136a is preferably placed closer to the proximal end side in comparison with the distal end of the main body 122, and the proximal end of the projected surface 136a is preferably placed closer to the distal end side in comparison with the proximal end of the main body 122 . The projected surface 136a is preferably formed as an obtuse shape in which the contact area with the living tissue is large, in order to prevent a concentration of energy to the projected surface 136a of the blade 136.

However, the projected surface 136a of the blade 136 may be acute, as described in the third embodiment. As the projected surface 136a becomes acute, energy can be concentrated onto the projected surface 136a of the blade 136 even with the same power, and early sealing of living tissue around the center in the width direction β can be conducted. In addition, as the projected surface 136a becomes acute, greater pressure can be added to living tissue. Accordingly, it is easily imagined that incision of living tissue by applying heat energy can be expedited.

The second treatment piece 104 includes the second jaw (lower-side jaw) 152 and a second holding body 154. The second jaw 152 preferably includes a material which has electrical insulation properties and heat resistance.

A plurality of pins 156 are disposed between the second jaw 152 and the second holding body 154, in parallel to the width direction β. Accordingly, the second jaw 152 is fixed to the second holding body 154 by the pins 156. The second jaw 152 protects a reverse side of the second holding body 154.

The second holding body 154 includes a main body 162 supported by the second jaw 152, a pair of holding bodies 164 fixed to the main body 162, and a receiving portion 166 formed between the pair of holding bodies 164. The receiving portion 166 has a concave shape in this embodiment.

The main body 162 is formed of a material which has electrical insulation properties, heat resistance, and resilience. The main body 162 is preferably formed of a material which has low thermal conductivity. Accordingly, it is possible to prevent heat energy transferred from the first holding section 124 when the heating element 126 generates heat, from moving toward the second jaw 152 to the greatest extent possible.

The receiving portion 166 extends in the longitudinal direction α. The receiving portion 166 is preferably formed to have the length equal to or slightly shorter than the main body 162 in the longitudinal direction α. The distal end of the receiving portion 166 is preferably placed closer to the proximal end side in comparison with the distal end of the main body 162, and the proximal end of the receiving portion 166 is preferably placed closer to the distal end side in comparison with the proximal end of the main body 162.

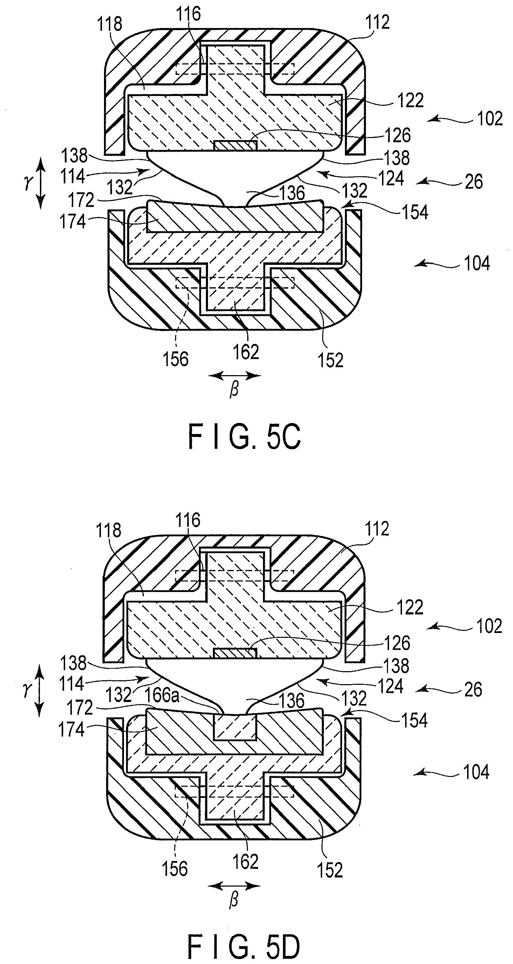

The main body 162 and the holding bodies 164 are formed to be longer in the longitudinal direction α than in the width direction β. The holding bodies 164 have the second holding surfaces 172 and a pair of second electrode portions (second high frequency electrode portions) 174. The pair of second electrode portions 174 are formed of, for example, a material having good conductivity such as copper, nickel alloy material, etc. In this embodiment, each of the pair of second electrode portions 174 has a second holding surface 172. The pair of second electrode portions 174 may be connected at the distal end and formed as an essentially U-shape on a virtual surface defined by the longitudinal direction α and the width direction β of the holding bodies 164 (see FIG. 5C), or may be separated by a distal end 166a of the receiving portion 166 (see FIG. 5D) . In the former case (FIG. 5C), the second electrode portions 174 are consecutive as an essentially U-shape on the surface of the main body 162. In the latter case (FIG. 5D), the second electrode portions 174 are not consecutive on the surface of the main body 162, but are connected inside the main body 162 by a lead, for example, so that the second holding surfaces 172 have the same potential. In either case, the second electrode portions 174 are formed to enclose the perimeter of the receiving portion 166.

Each of the pair of second holding surfaces 172 is formed as an inclined surface which is inclined relative to a virtual surface defined by the longitudinal direction α and the width direction β of the holding body 164. The second holding surfaces 172 are each formed as an inclined surface in which the side closer to the center in the width direction β is positioned lower than the side closer to the end portion (side surface 168) . The thickness (in the open/close direction γ) of the holding body 164 is formed to decrease toward the center in the width direction β to be away from the blade 136, which is used as the first electrode portion, and to increase toward the side surface 168 to apply suitable pressure to living tissue cooperatively with the first holding surfaces 132. The second holding surfaces 172 can set suitable pressure to be applied to living tissue in the width direction β by collaborating with the first holding surfaces 132.

In either case where the first and second treatment pieces 102 and 104 are opened or closed, the blade 136 and the second electrode portions 174 are maintained to be separated. Similarly, in either case where the first and second treatment pieces 102 and 104 are opened or closed, the first and second electrode portions 134 and 174 are maintained as being separated.

As shown in FIG. 1, the control section 14 includes a controller 182, a high frequency energy source 184, a heat energy source 186, a setting portion 188, and a memory 190. The setting portion 188 performs various settings relative to the high frequency energy source 184 and the heat energy source 186 . The setting portion 188 may adopt a keyboard or a touch panel, etc. The memory 190 stores items set by the setting portion 188 .

In addition, the setting portion 188 can discretionarily and respectively set an energy output timing for the high frequency energy source 184 and the heat energy source 186. For example, it is possible to set that after the high frequency energy source 184 initiates an output of energy, the heat energy source 186 initiates an output of energy, or that the high frequency energy source 184 initiates an output of energy at the same time as the heat energy source 186 outputs energy.

The high frequency energy source 184 can measure an impedance of the living tissue held between the electrode portions 134 and 174 over time, by means of the electrode portions 134 and 174. That is, the electrode portions 134 and 174 can be used as bi-polar type high frequency electrodes. In addition, the high frequency energy source 184 is preferably constant-power controlled by the controller 182 based on the impedance of the living tissue between the electrodes 134 and 174. A power P1 of the high frequency energy source 184 will be explained to be 40 W, for example, in this embodiment, but may be discretionarily set within a range from 30 W to 100 W, for example.

The heat energy source 186 can measure a resistance value of the heating element 126 at appropriate time intervals. In this embodiment, the controller 182 controls the heat energy source 186 such that the heating element 126 which is kept at constant temperature. Specifically, the controller 182 monitors the resistance value of the heating element 126, and controls a current and a voltage to be applied to the heating element 126 from the heat energy source 186 to obtain a desired resistance value. When energy is supplied to the heating element 126 from the heat energy source 186, the temperature T2 of the heating element 126 is set by the setting portion 188 to be within a range from 220 °C to 400 °C. Especially, the temperature T2 is preferably set within a range from 250 °C to 350 °C.

A temperature T0 (<T2) immediately before allowing the heating element 126 to generate heat may be room temperature or a temperature which is increased from room temperature to a degree not affecting living tissue (for example, 60 °C or lower), based on a monitor output. It is preferable that the temperature T0 is increased from room temperature by heat transmission from the living tissue.

Normally, a large amount of power is supplied to the heating element 126 from the heat energy source 186 to actively dehydrate living tissue (advanced dehydration) . However, if the moisture amount of living tissue becomes small, there is no need to perform further dehydration, and the power required for constant temperature control can be reduced. Accordingly, if living tissue is dehydrated by using high frequency energy beforehand, power to be supplied from the heat energy source 186 to the heating element 126 can be reduced.

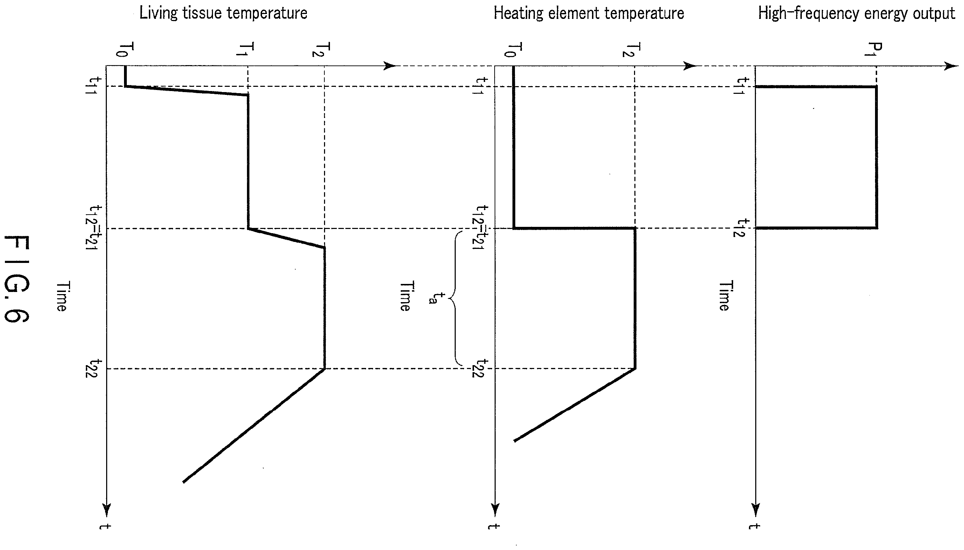

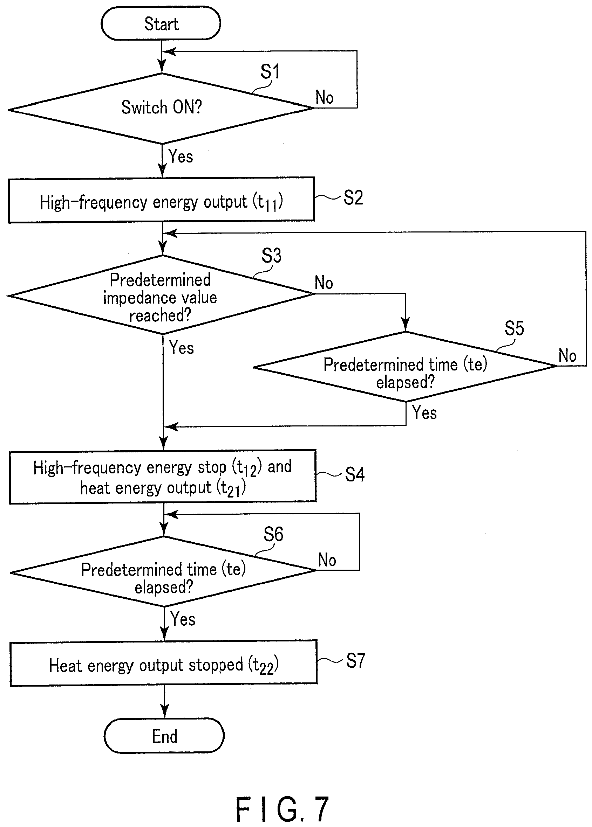

Next, the procedure of the treatment system 10 according to the present embodiment will be described with reference to FIGS. 6 and 7.

As shown in reference to FIG. 6, the treatment instrument 12 according to the embodiment seals (coagulates) the protein of living tissue of the treatment target by denaturing and dehydrating by using high frequency energy, and incises the sealed portion by supplying heat energy to the sealed portion to cause advanced denaturation of protein (structural change in a linear combination of protein), and applying pressure from the blade 36 to the sealed portion. The state of supplying high frequency energy and heat energy to living tissue can be discretionarily set by the setting portion 188.

A user suitably sets the state relative to the high frequency energy source 184 and the heat energy source 186 at the setting portion 188. For example, the user sets a maximum treatment time, for example, within a range approximately from 5 seconds to 10 seconds. The setting portion 188 sets a setting output (power) of the heat energy source, and sets the second temperature T2 of the heat energy source 186 to be within a range from 220 °C to 400 °C.

1. The user holds the operation portion 22 and brings the treatment portion 26 to face a treatment target in a body cavity. If there is a difficulty in holding living tissue by the treatment portion 26, the rotating lever 42 is rotated to rotate the treatment portion 26 relative to the distal end of the tubular body 24. The movable handle 34 of the operation portion 22 is appropriately operated to hold living tissue to be treated in a state where the treatment portion 26 faces the living tissue.

At this time, the living tissue is in contact between the pair of first holding surfaces 132 and the pair of second holding surfaces 172, which are elongated in the longitudinal direction α. Similarly, the living tissue is in contact between the projected surface 136a of the blade 136 and the receiving portion 166, which are elongated in the longitudinal direction α. The greatest pressure is applied to a portion of the living tissue sandwiched between the projected surface 136a of the blade 136 and the receiving portion 166. In addition, the distance between the holding surfaces 132 and 172 decreases, and the pressure increases as the holding surfaces 132 and 172 come closer to the blade 136 in the width direction β. The distance between the holding surfaces 132 and 172 increases, and the pressure decreases as the holding surfaces 132 and 172 are separated from the blade 136 in the width direction β. Accordingly, it is possible to locally increase pressure to living tissue when the living tissue is held between the first and second treatment pieces 102 and 104, depending on the shape of the projected surface 136a of the blade 136.

When a pedal (not shown in the drawings) of the footswitch 16 is stepped on (Step S1 in FIG. 7), the controller 182 controls the high frequency energy source 184 and the heat energy source 186 to output energy in order given, based on the setting by the setting portion 188.

As shown in FIG. 6, if a signal is input from the footswitch 16, the controller 182 controls the high frequency energy source 184 to output high frequency energy to the living tissue through the first and second electrodes 134 and 174 with a constant power P1 from t11 seconds after the receipt of the signal (Step S2 in FIG. 7) . The first and second electrode portions 134 and 174 collaboratively raise the temperature of the living tissue, held between the first and second electrode portions 134 and 174 by the pair of first holding surfaces 132 and the pair of second holding surfaces 172, from the temperature T0 to the temperature (first temperature) T1 that is a temperature between 60 °C and 120 °C, by heat, for example by Joule heat, generated at the held living tissue by high frequency energy applied to the living tissue. The temperature T1 is a temperature sufficient at least to denature protein (structural change of secondary, tertiary combination of protein), to dehydrate tissue, and to seal the tissue. It is easily understood for a person with ordinary skill in the art that Joule heat generated at the held living tissue by high frequency energy is difficult to increase up to the second temperature T2 (incision temperature of approximately from 220 °C to 400 °C), which is higher than the first temperature T1 (coagulate temperature).

In this case, denaturation of protein and tissue dehydration proceeds in the living tissue between the first and second holding surfaces 132 and 172, and the living tissue between the first and second holding surfaces 132 and 172 is sealed.

The blade 136 also functions as part of the electrode portion 134. Accordingly, the temperature of the living tissue between the projected surface 136a of the blade 136 and the second holding surfaces 172 increases to a temperature sufficient to denature protein and dehydrate tissue, and the living tissue is sealed. The living tissue held (contacted) by the first and second holding portions 124 and 164 is sealed to be a sheet-like shape having a suitable area along the longitudinal direction α and the width direction β.

In this case, the greatest pressure is applied to a portion of the living tissue sandwiched between the projected surface 136a of the blade 136 and the receiving portion 166, and the blade 136 and the second holding surfaces 172 are close to each other. Accordingly, the energy density of the portion of the living tissue contacted between the projected surface 136a of the blade 136 becomes greatest, and the portion is easy to be sealed. In addition, the distance between the holding surfaces 132 and 172 decreases, and the pressure applied to the living tissue increases, as the holding surfaces 132 and 172 come closer to the blade 136. Thus, the living tissue held between the holding surfaces 132 and 172 becomes easier to be sealed as the living tissue comes closer to the projected surface 136a of the blade 136.

In the case where the distal end of the second electrode portion 174 of the second treatment piece 104 is consecutive as an essentially U-shape on the surface of the main body 162, as shown in FIG. 5C, the sealing area of the living tissue is formed as an essentially U-shape. In the case where the distal end is not consecutive on the surface of the main body 162, as shown in FIG. 5D, the sealing area of the living tissue may be formed separately.

If denaturation of protein and tissue dehydration proceeds in the living tissue between the first and second holding surfaces 132 and 172, and the projected surface 136a of the blade 136 and the second holding surface 172, by the output from the high frequency energy source 184, the impedance measured through the living tissue between the electrode portions 134 and 174 increases. The controller 182 can detect that an impedance value measured between the electrode portions 134 and 174 increases to a predetermined value set by the setting portion 188 for a time shorter than a predetermined time te (for example, several seconds) (>t12), by the output from the high frequency energy source 184 (step S3) . At time t12 before the predetermined time te has elapsed, the output from the high frequency energy source 184 is stopped, and energy is output from the heat energy source 186 (t21=t12) to allow the heating element 126 to generate heat, for example, of 250 °C to heat the sealed living tissue between the projected surface 136a of the blade 136 and the receiving portion 166 to the temperature T2 sufficient for incision (step S4). That is, the control section 14 switches from the state where energy is supplied to the first and second electrode portions 134 and 174 to increase the temperature of the living tissue to the first temperature T1 or to maintain the temperature T1, to the state where energy is supplied to the heating element 126 to heat the first holding surfaces 132 to the second temperature T2 (>first temperature T1).

In this embodiment, the output from the high frequency energy source 184 is stopped at time T12; however, it is possible to continue the output, and it is preferable to continue outputting low power not affecting the treatment of the living tissue (for example, approximately several watts) as the monitor output. In the specification, "the output from the high frequency energy source 184 is stopped at time T12" includes the case where the monitor output is continued, in addition to the case where the output is actually stopped. At time t22 described later, the monitor output from the high frequency energy source 184 is completely stopped. By the monitor output from the high frequency energy source 184, the impedance of the living tissue can be continuously measured.

The controller 182 can detect that the impedance measured between the electrode portions 134 and 174 has not increased to the predetermined value set by the setting portion 188 within the predetermined time te (for example, several seconds), by the output from the high frequency energy source 184 (step S3) . At this time, even if the impedance does not increase up to the predetermined value within the predetermined time te from the time t11 of output initiation of the high frequency energy source 184 (step S5), the output from the high frequency energy source 184 is stopped, and energy is output from the heat energy source 186 to allow the heating element 126 to generate heat for example of 250 °C to increase the temperature to the temperature T2 sufficient for incision.

Even if the living tissue is sealed by the high frequency energy, the greatest pressure is maintained to be applied to the living tissue sandwiched between the projected surface 13 6a of the blade 136 and the receiving portion 166.

In this state, heat from the heating element 126 is transmitted to the first holding surfaces 132 and the projected surface 136a of the blade 136 through the holding body 124 having good thermal conductivity. In this case, the projected surface 136a of the blade 136 having an obtuse shape applies greater pressure to the sealed living tissue and heats the living tissue further to approximately 250 °C (temperature T2), in comparison with the first holding surfaces 132. Thus, the projected surface 136a of the blade 136 applies pressure to the living tissue while heating the living tissue. The temperature T2 is a temperature sufficient for advanced denaturation of protein (structural change in a linear combination of protein). Accordingly, the projected surface 136a of the blade 136 applies pressure to the living tissue held between the first and second holding surfaces 132 and 172, and incises the living tissue. If the projected surface 136a of the blade 136 holds the living tissue and applies high frequency energy and heat energy to the living tissue while the projected surface 136a of the blade 136 is brought into contact with a position of the living tissue to be incised, the living tissue is incised at the position that is in contact with the projected surface 136a of the blade 136. The holding surfaces 132 and 172 that are placed at both sides of the projected surface 13 6a of the blade 13 6 do not receive pressure sufficient for incision, and accordingly, a high sealing force is accomplished by advanced dehydration.

That is, the control section 14 can supply energy to the first and second electrode portions 134 and 174, heat the living tissue held between the first and second holding surfaces 132 and 172 to the first temperature T1 by Joule heat to seal the living tissue, and supply energy to the heating element 126 provided to the first holding surfaces 132 to heat the first holding surfaces 132 so that the temperature of the living tissue held between the first and second holding surfaces 132 and 172 is increased to the second temperature T2, which is higher than the first temperature T1. Accordingly, the living tissue can be incised by the projected surface 136a of the blade 136 while a high tissue sealing force is accomplished around the projected surface 136a.

Then, with regard to the heat energy source 186, it is determined whether a predetermined time ta (t22-t21 is constant) has elapsed from the output initiation (time t21) (step S6). After the predetermined time ta (several seconds) has elapsed from the initiation of heat energy output, the energy output is stopped (step S7). In this state, a series of treatments using the treatment system 10 is temporarily terminated. By the termination of the treatment, the temperature of the heating element 126 is gradually decreased to room temperature. The time required for the series of treatment may vary according to the change in time t12.

The termination of the treatment may be displayed on a monitor, etc. (not shown in the drawings) connected to the control section 14, or may be informed to a user by a sound output from the control section 14.

In the above, an example where the treatment system 10 performs automatic control is explained; however, a user may perform an output operation such as initiation and termination of output by using the footswitch 16. In addition, a user may perform an output operation for each of high frequency energy and heat energy by using the footswitch 16.

If required, the movable handle 34 is suitably operated so that the treatment portion 26 suitably holds the distal end side of the incised living tissue, and the similar treatment is performed. Accordingly, the living tissue can be incised while being sealed.

As explained above, the treatment system 10 according to the present embodiment accomplishes the following.

The treatment system 10 and the treatment instrument 12 according to the present embodiment perform sealing (coagulating) of living tissue and incising the sealed portion immediately after sealing by one action, which is stepping on the footswitch 16 to turn a switch on, in the state where the treatment portion 26 is holding the living tissue. Accordingly, if the footswitch 16 is stepped on while the treatment portion 26 of the treatment instrument 12 is holding living tissue, a series of operations of sealing (coagulating) of the living tissue to be treated and incising the sealed portion is realized by one action. That is, when repetitively performing operations of reliably sealing (coagulating) living tissue with high frequency energy, and incising the living tissue with heat energy, the treatment instrument 12 can incise a central part of the living tissue to be treated (to be incised) while sealing the periphery of the portion to be incised only by holding the living tissue to be treated and turning on a switch. Accordingly, the time required for incising living tissue while being sealed can be shortened by using the treatment system 10 and the treatment instrument 12.

In addition, the heating element 126 generates heat of the temperature T2 of approximately 250 °C or 300 °C which is sufficient for incision of living tissue, and heat from the heating element 126 can be applied to living tissue through the projected surface 136a of the blade 136 which is made of a material having good thermal conductivity.

The gap 118 is defined between the first jaw 112 and the first holding body 114 of the main body 122. Accordingly, it can be prevented to transmit heat from the main body 122 to the first jaw 112. In addition, even if the treatment portion 26 is unintentionally brought into contact with living tissue when the treatment portion 26 is moved while the first and second treatment pieces 102 and 104 are closed, adding heat energy to the living tissue due to heat from the heating element 126 can be prevented to the greatest extent possible. Thus, according to the treatment instrument 12 of the present embodiment, the temperature outside the treatment portion 26 can be controlled.

In addition, the treatment portion 26 of the treatment instrument 12 according to the embodiment includes the rotating shaft 108 which is capable of suitably rotating the treatment portion 26. Thus, unlike a probe used for treatment using ultrasonic vibration, it is not necessary for the treatment portion 26 to be rigid from the proximal end to the distal end, and may have a shape that facilitates accessibility to a treatment target.

In the treatment portion 26 of the treatment instrument 12 according to the embodiment, the high frequency electrode portions 134 and 174 and the heating element 126 output energy. The high frequency electrode portions 134 and 174 and the heating element 126 can be formed in an appropriate shape. Accordingly, the high frequency electrode portions 134 and 174 and the heating element 126 have a degree of freedom in shape. For example, the main body 122 and the holding body 124, i.e. , the main body 122, the electrode portion 134 and the blade 136, may be formed as an arc-shape from the proximal end to the distal end along the longitudinal direction α of the holding body 114, or formed by suitably bending into an S-shape. Similarly, the main body 162, the electrode portion 174 and the receiving portion 166 may be formed as an arc-shape from the proximal end to the distal end along the longitudinal direction α of the holding body 154, or formed by suitably bending as an S-shape. Thus, the first and second treatment pieces 102 and 104 are not limited to the shape shown in FIGS. 2A, 2B, 5A and 5B, but may be discretionarily shaped. By suitably forming the treatment portion 26 of the treatment instrument 12, the treatment portion 26 is adaptable to a variety of treatments.

In an example shown in FIG. 6, the energy output from the high frequency energy source 184 is stopped at the same time that the energy output from the heat energy source 186 is initiated. Alternatively, it is possible that the energy output from the high frequency energy source 184 is initiated at the same time that the energy output from the heat energy source 186 is initiated. When output from the high frequency energy source 184 and the heat energy source 186 is performed simultaneously during treatment, a user sets the output of the high frequency energy source 184 to be a range from 40 W to 120 W, by means of the setting portion 188. The temperatures T1 and T2 of the heating element 126 may be discretionarily set within a range from room temperature to 400 °C. The temperature for treatment using heat energy is preferably set, for example, between 60°C and 400°C, especially, between approximately 250 °C and 350 °C, for incision.

In an example shown in FIG. 6, the energy output from the high frequency energy source 184 is stopped at the same time that the energy output from the heat energy source 186 is initiated. Alternatively, it is possible that the energy output from the heat energy source 186 is initiated while the energy output from the high frequency energy source 184 is performed.

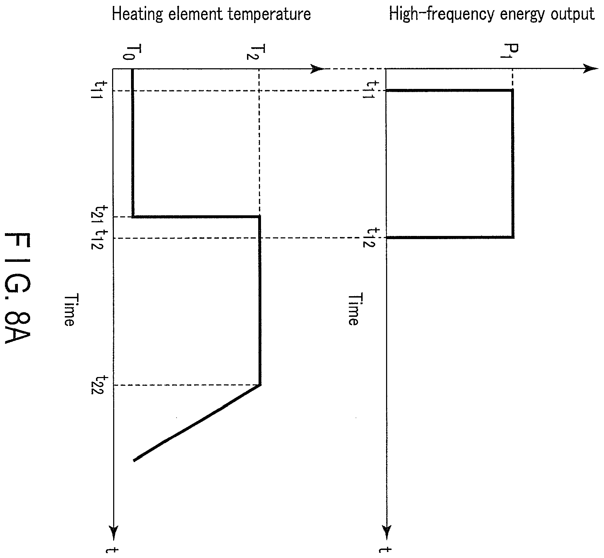

In this case, a predetermined impedance measured through living tissue between the electrode portions 134 and 174, which is used as a trigger to allow the heat energy source 186 to output energy, may be set to be lower than that explained above. Then, the energy output from the high frequency energy source 184 may be stopped when the impedance reaches the impedance value set as explained above. Thus, as shown in FIG. 8A, it is possible that the energy output from the heat energy source 186 is initiated at time t21, and the energy output from the high frequency energy source 184 is stopped at time t12. Alternatively, the high frequency energy may be continuously output. For example, the high frequency energy may be continuously output with a lower value than the output at time t12. That is, the output of the high frequency energy may be stopped, but may be continuously output with a suitable value. On the other hand, the energy output time from the heat energy source 186 may be set as a predetermined time, as shown in FIG. 6.

That is, the control section 14 can supply energy to the first and second electrode portions 134 and 174 to increase the temperature of living tissue to the first temperature T1, while supplying energy to the heating element 126 to heat the first holding surfaces 132 to the second temperature T2.

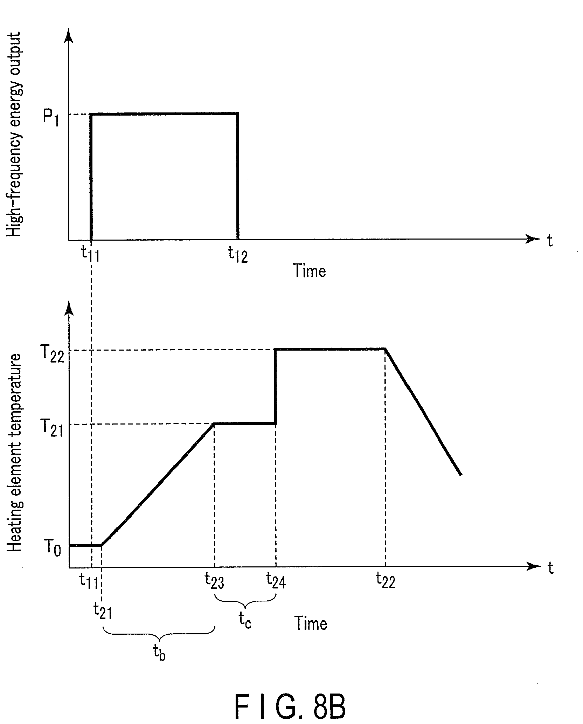

As shown in FIG. 8B, energy output from the heat energy source 186 may be initiated at time t21 that is immediately after time t11 when energy output from the high frequency energy source 184 is initiated. In this case, the heat energy source 186 is controlled to raise the temperature of the heating element 126 linearly from room temperature to T21 (approximately 200 °C) in several seconds tb (=t23-t21). The temperature T21 is not sufficiently high for incision of living tissue. Then, after the temperature T21 is maintained for several seconds tc (=t24-t23), the temperature is drastically raised to temperature (second temperature) T22 (for example, 300°C) . That is, the control section 14 can increase energy to be input to the heating element 126 in a stepwise manner. In other words, the control section 14 can increase the temperature of the heating element 126 to the second temperature T22 in a stepwise manner. If the impedance between the electrode portions 134 and 174 reaches a predetermined value while the energy output of the heat energy source 186 is controlled to maintain the temperature T21 for several seconds tc (=t24-t23) , the energy output from the high frequency energy source 184 is stopped.

In FIG. 8B, the temperature of the heating element 126 is maintained at the temperature T21 between time t23 and time t24 . However, it is of course possible that the temperature is linearly increased from room temperature to a target temperature (second temperature) T22, for example, 250°C or 300°C, in several seconds, and if the temperature reaches the target temperature T22, the temperature T22 is maintained until the treatment ends. The time t22 when the output from the heat energy source 186 is stopped may be set based on the time set at the setting portion 188.

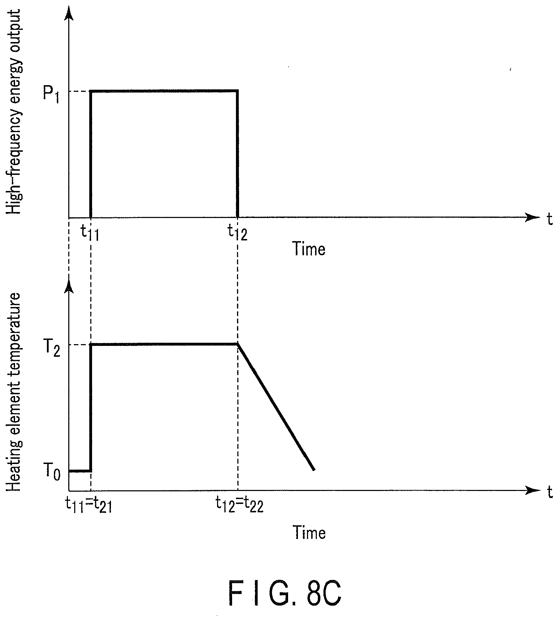

As shown in FIG. 8C, energy output from the high frequency energy source 184 and the heat energy source 186 may initiated simultaneously (t11=t12), and the energy output from the high frequency energy source 184 and the heat energy source 186 may be stopped simultaneously (t12=t22), based on the impedance or the treatment time which is used as a trigger. That is, it is possible to concurrently use a heating means when supplying high frequency energy to the heating element 126 to generate heat at time t11 and at time t22. In this case, the output from the heat energy source 186 is stopped at time t11 and at time t22, and accordingly, the monitor output from the high frequency energy source 184 is unnecessary after time t12 has elapsed.

The first and second holding surfaces 132 and 172 and the projected surface 136a are preferably subjected to surface processing such as coating to avoid living tissue becoming attached when energy is output from the high frequency energy source 184 and the heat energy source 186, while conductivity is maintained.

Thus, when treatment is advanced by one action, the control section 14 may supply energy to the first and second electrode portions 134 and 174 simultaneously to supply energy to the heating element 126, as shown in FIG. 8C. Alternatively, the control section 14 may supply energy to the heating element 126 after initiation of energy supply to the first and second electrode portions 134 and 174 as shown in FIGS. 6, 8A, and 8B. In either situation for energy supply, it is possible to perform treatment to seal a portion of living tissue to be sealed by heating the portion to be sealed to the first temperature T1, and to incise a portion of the living tissue to be incised, the portion to be incised which is inside of the portion to be sealed, by heating the portion to be incised to the second temperature T2 that is higher than the first temperature T1.

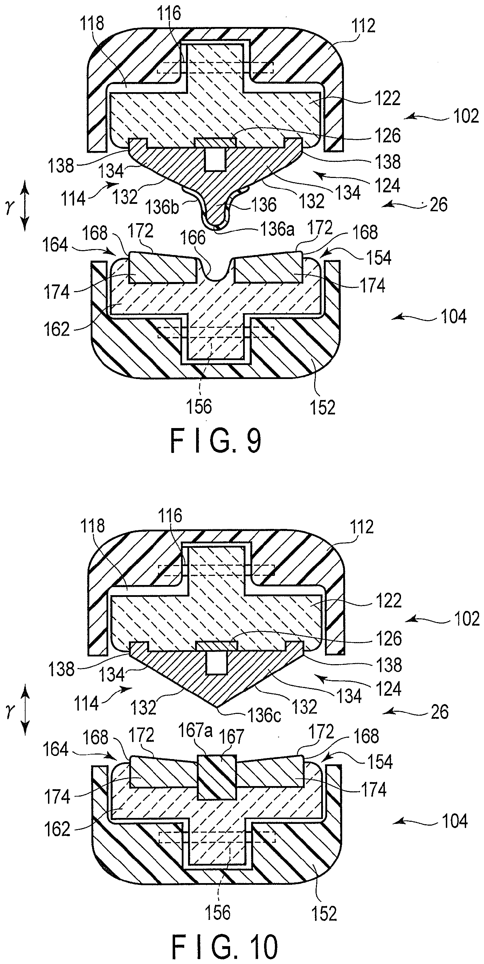

Next, the second embodiment will be explained with reference to FIG. 9. The present embodiment is a modification of the first embodiment, in which, to omit detailed explanations, the same symbols as those in the first embodiment will be applied whenever possible to the same members or the members with the same functions as those explained in the first embodiment.

In the second embodiment onward, it is assumed that the high frequency energy source and the heat energy source sequentially output energy in order as shown in the graph of FIG. 6 and the flowchart of FIG. 7. It is also preferable that treatment is performed by outputting energy to living tissue as shown in FIGS. 8A to 8C.

As shown in FIG. 9, a projected surface 136a is preferably covered with a coating 136b which has electrical insulation properties and heat resistance. The coating 136b is consecutively formed from the proximal end to the distal end along the longitudinal direction α. In this case, the coating 136b having electrical insulation properties blocks an energy flow from a high frequency energy source 184 between the projected surface 136a of a blade 136 and second electrode portions 174. Accordingly, a current is applied to living tissue mainly between the first electrode portions 134 and the second electrode portions 174. With this structure, the living tissue between first holding surfaces 132 and second holding surfaces 172 is sealed. Part of the living tissue which is brought into contact with the coating 136b of the blade 136 may be sealed by the effects of Joule heat.

The coating 136b gives electrical insulation properties to the projected surface 136a. However, it is more preferable that the coating 136b has a function of preventing adhesion of living tissue.

Next, the third embodiment will be explained with reference to FIG. 10. This embodiment is a modification of the first and second embodiments. To omit detailed explanations, same members or the members having the same functions as those of the members of the first and second embodiments are identified by the same reference symbols as those used for those embodiments whenever possible.

In the first embodiment, the projected surface 136a of the blade 136 has an obtuse shape. As shown in FIG. 10, the cross sectional surface of a projected surface 136a of a blade 136 is preferably formed as a V-shape. That is, the projected surface 136a of the blade 136 has an edge 136c from the proximal end to the distal end along the longitudinal direction α. In FIG. 10, first holding surfaces 132 are preferably formed as a plane or essentially plane consecutive relative to the projected surface 136a.

It is also preferable that a receiving portion 166 is formed between the pair of holding bodies, as shown in FIGS. 5A and 5B, similar to the first embodiment. As a modification of the receiving portion 166, a receiving portion 167 formed of an elastic body that is elastically deformable and has electrical insulation properties and heat resistance, such as a resin material or rubber material is fixed between a pair of second electrode portions 174, instead of the receiving portion 166 which has a concave shape. That is, the second electrode portions 174 are formed to enclose the perimeter of the receiving portion 167. The receiving portion 167 may be a block shape or a film shape if it has a surface 167a that receives the edge 136c.

The distance between the holding surfaces 132 and 172 decreases, and a pressure applied to living tissue increases as the holding surfaces 132 and 172 become closer to the blade 136 in the width direction β. Thus, the living tissue held between the holding surfaces 132 and 172 becomes easier to be sealed as the living tissue comes closer to the projected surface 136a of the blade 136.

The projected surface 136a of the blade 136 has a sharp edge 136c in comparison with the obtuse shape as explained in the first embodiment. Thus, an area which applies pressure to living tissue can be decreased so that the pressure can be focused on the decreased area. Accordingly, the speed of incision at a position of living tissue which is pressed by the edge 136c can be increased in comparison to when using the obtuse projected surface 136a, as explained in the first embodiment.

In addition, this embodiment is a modification of the second embodiment, and the aforementioned coating 136b is preferably provided to the edge 136c. Although it depends on the area of the coating 136b, it is possible to prevent a concentration of current density to the edge 136c when sealing the living tissue by using high frequency energy. Accordingly, it is possible to seal living tissue brought into contact between the holding surfaces 132 and 172 by generating Joule heat.

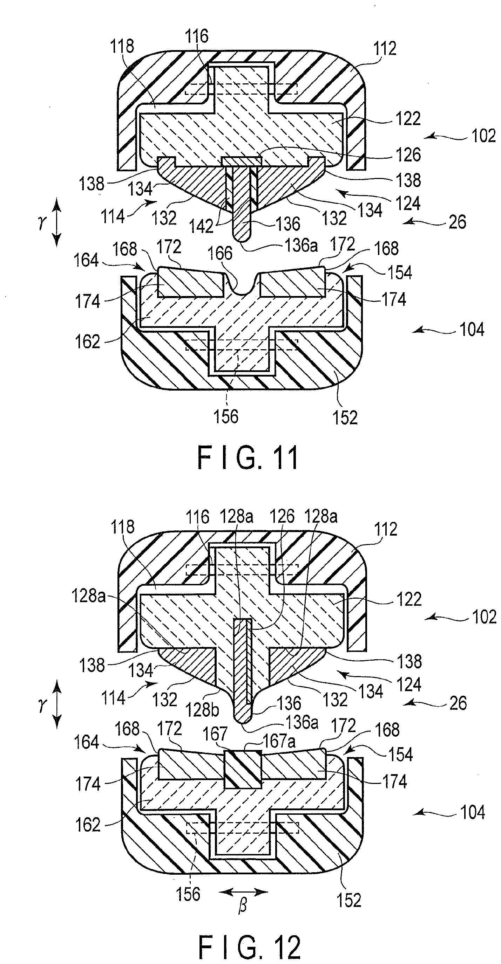

Next, the fourth embodiment will be explained with reference to FIG. 11. This embodiment is a modification of the first to third embodiments. To omit detailed explanations, the same members or the members having the same functions as those of the members of the first to third embodiments are identified by the same reference symbols as those used for those embodiments whenever possible.

As shown in FIG. 11, a first holding section 124 according to the embodiment is formed as a two-part or three-part body. An electrode portions 134 and a blade 136 of the first holding section 124 is separately formed, instead of integrally formed. The electrode portions 134 have a ring shape, namely, may be formed as a one-part body, or as a two-part body, if the same potential is reliably maintained. For example, the electrode portions 134 may be formed of copper alloy, and the blade 136 may be formed of nickel alloy. That is, it is preferable that the electrode portions 134 and the blade 136 are formed of materials that are mutually different in thermal conductivity or conductivity.

An insulation plate 142 that has electrical insulation properties and heat resistance is disposed between the electrode portions 134 and blade 136. Accordingly, the blade 136 is electrically separated from the electrode portions 134.

The blade 136 is preferably not electrically connected to a high frequency energy source 184. In this case, even if the high frequency energy source 184 outputs energy, Joule heat is not generated to living tissue between the blade 136 and second electrode portions 174. Thus, a part of the living tissue between the first and second holding surfaces 132 and 172 is sealed by the output of energy from the high frequency energy source 184.

Accordingly, it is possible to prevent energy from being focused on the living tissue between the blade 136 and the second electrode portions 174 when the high frequency energy source 184 outputs energy.

If the heating element 126 generates heat, heat energy can be transmitted to living tissue through the blade 136. Accordingly, living tissue inside the portion held and sealed between the pair of the holding surfaces 132 and 172 can be incised due to the pressure and the heat energy of the projected surface 136a of the blade 136.

It is preferable that the heating element 126 is in contact not only with the blade 136, but also with the electrode portions 134. In this case, heat energy can be transmitted to living tissue through the electrode portions 134. Accordingly, while living tissue between the holding surfaces 132 and 172 is dehydrated, the living tissue can be incised by the projected surface 136a which applies higher pressure in comparison with the other portions.

Next, the fifth embodiment will be explained with reference to FIG. 12. This embodiment is a modification of the first to fourth embodiments . To omit detailed explanations, the same members or the members having the same functions as those of the members of the first to fourth embodiments are identified by the same reference symbols as those used for those embodiments whenever possible.

As shown in FIG. 12, a main body 122 includes a plurality of recesses 128a. The recesses 128a respectively include electrode portions 134, a blade 136, and a heating element 126 inside thereof. An inclined surface 128b formed as a part of the main body 122 is formed between a projected surface 136a of the blade 136 and each of a pair of holding surfaces 132 formed in the electrode portions 134. Thus, in this embodiment, the inclined surface 128b, which is a part of the main body 122 having electrical insulation properties and heat resistance, functions as a holding surface. In this embodiment, the inclined surface 128b, which has electrical insulation properties and heat resistance, is a part of the holding surfaces 132.

The heating element 126 transmits heat energy relative to the blade 136 in the width direction, and transmits heat to the projected surface 136a of the blade 136.

Part of a main body 162 between second holding surfaces 172 is formed as a flat surface in this embodiment. Even in this structure, living tissue can be sealed and incised, as explained in the first embodiment.

Although not shown in the drawings, it is preferable that a coating 136b is formed on the projected surface 136a of the blade 136.

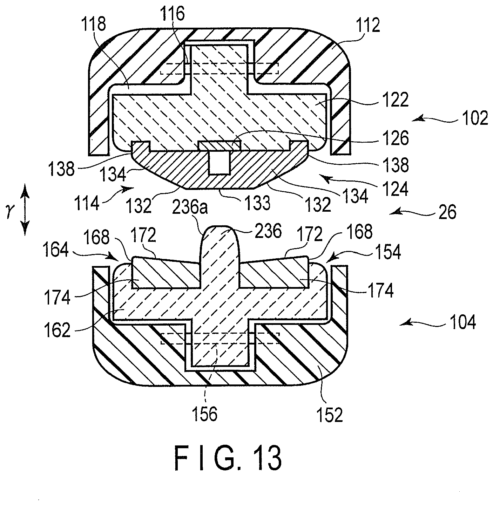

Next, the sixth embodiment will be explained with reference to FIG. 13. This embodiment is a modification of the first to fifth embodiments. To omit detailed explanations, the same members or the members having the same functions as those of the members of the first to fifth embodiments are identified by the same reference symbols as those used for those embodiments whenever possible.

As shown in FIG. 13, in this embodiment, a blade 136 is not provided to a first treatment piece 102, and a blade 236 is provided to a second treatment piece 104. The blade 236 is preferably formed of a material which has electrical insulation properties and heat resistance, such as PTFE, for example. The blade 236 may be formed integrally with the main body 162, or independently from the main body 162.

A receiving portion (plane portion) 133 which is a plane or a smooth curved surface, is formed between a pair of holding surfaces 132 of the first treatment piece 102. In a similar manner as the first holding surfaces 132, the receiving portion 133 is formed of a material which has good conductivity and good thermal conductivity, such as copper alloy material, for example. The receiving portion 133 is preferably formed integrally with the first holding surfaces 132. It is also preferable that the receiving portion 133 is coated, in a similar manner by a coating 136b as explained in the second embodiment.

On the other hand, the blade 236 of the second treatment piece 104 has a projected surface 236a that is formed between a pair of electrode portions 174 to project toward the receiving portion 133. The projected surface 236a is formed from the proximal end to the distal end along the longitudinal direction α of the main body 162 of the second holding body 164. The projected surface 236a may be an obtuse shape like the projected surface 136a explained in the first embodiment, or have an edge like the projected surface 136a explained in the third embodiment.

As explained in the first embodiment, when living tissue is held between the first and second treatment pieces 102 and 104, pressure is applied to the living tissue by the projected surface 236a from a surface opposite to the projected surface 136a explained in the first embodiment. In this state, if energy is supplied to the living tissue from a high frequency energy source 184, the living tissue between the electrode portions 134 and 174 is sealed.

When a part which is in contact with the receiving portion 133 and receives pressure by the projected surface 236a is incised, the living tissue can be further dehydrated by the applied pressure and heat transmitted from the receiving portion 133. Accordingly, the projected surface 236a of the blade 236 applies pressure to the living tissue held between the first and second holding surfaces 132 and 172, and incises the living tissue.

While some embodiments have been specifically described with reference to the drawings, this invention is not limited to the embodiments described above, and embraces all implementations conducted without departing from the invention, as defined by the appended claims.

### Reference Signs List

10...treatment system, 12...treatment instrument, 26...treatment portion, 102...first treatment piece, 104...second treatment piece, 106...opening-and-closing shaft, 108...rotating shaft, 112...first jaw, 114...holding body, 118...gap (air layer), 122...first main body, 124...first holding portion, 126...heater, 132...first holding surface, 134...first high frequency electrode portion, 136...blade, 136a...projected surface, 138...side surface, 152...second jaw, 154...second holding body, 162...second main body, 164...second holding portion, 166...receiving portion, 168...side surface, 172...second holding surface, 174...second high frequency electrode portion, a...longitudinal direction, β...width direction, γ...open/close direction.

## Claims

1. A treatment instrument (12) comprising:
a first holding surface (132) which includes a first high frequency electrode portion (134);
a pair of second high frequency electrode portions (174) which includes a second holding surface (172) opposed to the first holding surface (132), configured to hold living tissue cooperatively with the first holding surface (132), configured to supply high frequency energy to the living tissue, and configured to raise a temperature of the living tissue held between the first high frequency electrode portion (134) and the second high frequency electrode portion (174) to a first temperature (T1) sufficient at least to denature protein, to seal the living tissue by heat cooperatively with the first high frequency electrode portion (134);
a blade (136) which is provided on the first holding surface (132), which includes a projected surface (136a) projecting from the first holding surfaces (132), which is formed at a center of the first holding surface (132) in a width direction (β), and which is configured to apply pressure to the living tissue held between the first and second holding surfaces (132, 172) by the projected surface (136a); and
a heater (126) which is configured to generate heat and supply the heat to the first high frequency electrode portion (134), wherein the heater is capable of heating the first holding surface (132) to a second temperature (T2) higher than the first temperature (T1) and sufficient to incise the living tissue by the blade (136), upon receipt of energy supplied at the same time as or after energy supply to the first and second high frequency electrode portions (134, 174);
wherein the second holding surface (172) includes a receiving portion (166) having electrical insulation properties, and is brought into contact with the projected surface (136a) of the blade (136); and
wherein the receiving portion (166) has a concave shape, a plane or smooth curved surface which is provided between the pair of second high frequency electrode portions (174).

2. The treatment instrument (12) according to claim 1, wherein the projected surface (136a) has electrical insulation properties.

3. The treatment instrument (12) according to claim 1, wherein the projected surface (136a) is integrally formed with the first holding surface (132).

4. The treatment instrument (12) according to claim 1, wherein
the first holding surface (132) and the second holding surface (172) are defined by a longitudinal direction (α) in which the projected surface (136a) extends, a width direction (β) which is orthogonal to the projected surface (136a), and an open/close direction (γ) in which the first holding surface (132) and the second holding surface (172) are opened or closed, and
a distance between the first holding surface (132) and the second holding surface (172) increases as the first holding surface (132) and the second holding surface (172) are separated to the projected surface (136a) in the width direction (β).

5. The treatment instrument (12) according to claim 1, wherein the first holding surface (132) is smoothly continuous to the projected surface (136a).

6. The treatment instrument (12) according to claim 1, wherein:
the first high frequency electrode portion (134) includes a first inclined surface that is inclined from a center to a side portion of the first holding surface (132) in the width direction (β),
the second high frequency electrode portions (174) include a second inclined surface that is opposed to the first inclined surface and has an inclination different from the first inclined surface, and
when the living tissue is held between the first inclined surface and the second inclined surface, a distance between the first inclined surface and the second inclined surface increases from the center to the side portion of the first holding surface (132) in the width direction (β).

7. A treatment system (10) comprising:
the treatment instrument (12) recited in claim 1; and
a controller (182) which is configured to implement supplying energy to each of the first high frequency electrode portion (134), the second high frequency electrode portion (174) and the heater (126) of the treatment instrument (12).

8. The treatment system (10) according to claim 7, wherein
the controller (182) is configured to implement:
supplying the energy to the first high frequency electrode portion (134) and the second high frequency electrode portion (174) such that a temperature of the living tissue held between the first holding surface (132) and the second holding surface (172) is raised to a first temperature (T1) sufficient to seal the living tissue by heat generated by the energy and the living tissue is sealed; and
supplying the energy to the heater (126) such that heat of the heater (126) is configured to heat the first holding surface (132) to a second temperature (T2) higher than the first temperature (T1) and sufficient to excise the living tissue held between the first holding surface (132) and the second holding surface (172) and the projected surface (136a) is configured to excise the living tissue, while increasing the temperature of the living tissue from the first temperature (T1) to the second temperature (T2).

9. The treatment system (10) according to claim 7, wherein a first temperature (T1) sufficient to seal the living tissue by heat generated by the energy is set within a range from 60 °C to 200 °C, and a second temperature (T2) higher than the first temperature (T1) and sufficient to excise the living tissue is set within a range from 220 °C to 400 °C in the controller (182).

10. The treatment system (10) according to claim 7, wherein the controller (182) is configured to implement:
raising the temperature of the living tissue held between the first holding surface (132) and the second holding surface (172) to a first temperature (T1) by heat generated at the living tissue by the energy supplied to the first high frequency electrode portion (134) and the second high frequency electrode portion (174), and the energy of the heater (126) which is configured to heat the first holding surface (132) such that the living tissue is sealed; and
increasing the energy output to the heater (126) in comparison with a case of heating the living tissue to the first temperature (T1) to heat the first holding surface (132) to a second temperature (T2) higher than the first temperature (T1) and sufficient to incise the living tissue held between the first holding surface (132) and the second holding surface (172) such that the living tissue is incised by the projected surface (136a).

11. The treatment system (10) according to claim 7, wherein the controller (182) is configured to implement: switching from a state where the energy is supplied to the first high frequency electrode portion (134) and the second high frequency electrode portion (174) to increase the temperature of the living tissue to a first temperature (T1) sufficient to seal the living tissue to a state where the energy is supplied to the heater (126) to heat the first holding surface (132) to a second temperature (T2).

12. The treatment system (10) according to claim 7, wherein the controller (182) is configured to implement: supplying the energy to the heater (126) to heat the first holding surface (132) to a second temperature (T2) higher than the first temperature (T1) and sufficient to excise the living tissue while supplying the energy to the first high frequency electrode portion (134) and the second high frequency electrode portion (174) to increase the temperature of the living tissue to a first temperature (T1) sufficient to seal the living tissue.

13. The treatment system (10) according to claim 7, wherein the controller (182) is configured to implement constant temperature control to the heater (126).

14. The treatment system (10) according to claim 7, wherein the controller (182) is configured to implement increasing a temperature of the heater (126) to a second temperature (T2) sufficient to excise the living tissue in a stepwise manner.

## Patentansprüche

1. Behandlungsinstrument (12), das umfasst:
eine erste Haltefläche (132), die einen ersten Hochfrequenz-Elektrodenabschnitt (134) enthält;
ein Paar zweiter Hochfrequenz-Elektrodenabschnitte (174), die eine zweite Haltefläche (172) gegenüber der ersten Haltefläche (132) aufweisen, die so konfiguriert sind, dass sie lebendes Gewebe zusammenwirkend mit der ersten Haltefläche (132) halten, die so konfiguriert sind, dass sie dem lebenden Gewebe Hochfrequenzenergie zuführen, und die so konfiguriert sind, dass sie eine Temperatur des lebenden Gewebes, das zwischen dem ersten Hochfrequenz-Elektrodenabschnitt (134) und dem zweiten Hochfrequenz-Elektrodenabschnitt (174) gehalten wird, auf eine erste Temperatur (T1) anheben, die ausreicht, um zumindest Protein zu denaturieren, um das lebende Gewebe durch Wärme zusammenwirkend mit dem ersten Hochfrequenz-Elektrodenabschnitt (134) zu versiegeln;
eine Klinge (136), die an der ersten Haltefläche (132) vorgesehen ist, die eine vorstehende Fläche (136a) aufweist, die von der ersten Haltefläche (132) vorsteht, die in einer Mitte der ersten Haltefläche (132) in einer Breitenrichtung (β) ausgebildet ist, und die so konfiguriert ist, dass sie Druck auf das lebende Gewebe ausübt, das zwischen der ersten und der zweiten Haltefläche (132, 172) durch die vorstehende Fläche (136a) gehalten wird; und
ein Heizelement (126), das so konfiguriert ist, dass es Wärme erzeugt und die Wärme dem ersten Hochfrequenz-Elektrodenabschnitt (134) zuführt, wobei das Heizelement in der Lage ist, die erste Haltefläche (132) auf eine zweite Temperatur (T2) zu erwärmen, die höher ist als die erste Temperatur (T1) und ausreicht, um das lebende Gewebe durch die Klinge (136) einzuschneiden, wenn es Energie empfängt, die zur gleichen Zeit wie oder nach der Energiezufuhr zu dem ersten und dem zweiten Hochfrequenz-Elektrodenabschnitt (134, 174) zugeführt wird;
wobei die zweite Haltefläche (172) einen Aufnahmeabschnitt (166) mit elektrischen Isolationseigenschaften aufweist und in Kontakt mit der vorstehenden Fläche (136a) der Klinge (136) gebracht wird; und
wobei der Aufnahmeabschnitt (166) eine konkave Form, eine ebene oder glatt gekrümmte Oberfläche aufweist, die zwischen dem Paar von zweiten Hochfrequenz-Elektrodenabschnitten (174) vorgesehen ist.

2. Behandlungsinstrument (12) nach Anspruch 1, wobei die vorstehende Oberfläche (136a) elektrische Isolationseigenschaften aufweist.

3. Behandlungsinstrument (12) nach Anspruch 1, wobei die vorstehende Fläche (136a) einstückig mit der ersten Haltefläche (132) ausgebildet ist.

4. Behandlungsinstrument (12) nach Anspruch 1, wobei
die erste Haltefläche (132) und die zweite Haltefläche (172) durch eine Längsrichtung (α), in der sich die vorstehende Fläche (136a) erstreckt, eine Breitenrichtung (β), die orthogonal zu der vorstehenden Fläche (136a) ist, und eine Öffnungs-/Schließrichtung (γ), in der die erste Haltefläche (132) und die zweite Haltefläche (172) geöffnet oder geschlossen sind, definiert sind, und
ein Abstand zwischen der ersten Haltefläche (132) und der zweiten Haltefläche (172) zunimmt, wenn die erste Haltefläche (132) und die zweite Haltefläche (172) in der Breitenrichtung (β) von der vorstehenden Fläche (136a) getrennt sind.

5. Behandlungsinstrument (12) nach Anspruch 1, wobei die erste Haltefläche (132) nahtlos an die vorstehende Fläche (136a) anschließt.

6. Behandlungsinstrument (12) nach Anspruch 1, wobei:
der erste Hochfrequenz-Elektrodenabschnitt (134) eine erste geneigte Fläche aufweist, die von einer Mitte zu einem Seitenabschnitt der ersten Haltefläche (132) in der Breitenrichtung (β) geneigt ist,
die zweiten Hochfrequenz-Elektrodenabschnitte (174) eine zweite geneigte Fläche aufweisen, die der ersten geneigten Fläche gegenüberliegt und eine andere Neigung als die erste geneigte Fläche hat, und
wenn das lebende Gewebe zwischen der ersten geneigten Fläche und der zweiten geneigten Fläche gehalten wird, vergrößert sich ein Abstand zwischen der ersten geneigten Fläche und der zweiten geneigten Fläche von der Mitte zu dem Seitenabschnitt der ersten Haltefläche (132) in der Breitenrichtung (β).

7. Behandlungssystem (10), das umfasst:
das in Anspruch 1 beschriebene Behandlungsinstrument (12); und
ein Steuergerät (182), das so konfiguriert ist, dass es die Zufuhr von Energie zu jedem des ersten Hochfrequenz-Elektrodenabschnitts (134), des zweiten Hochfrequenz-Elektrodenabschnitts (174) und dem Heizelement (126) des Behandlungsinstruments (12) implementiert.

8. Behandlungssystem (10) nach Anspruch 7, wobei
das Steuergerät (182) so konfiguriert ist, dass implementiert:
Zuführen der Energie zu dem ersten Hochfrequenz-Elektrodenabschnitt (134) und dem zweiten Hochfrequenz-Elektrodenabschnitt (174), so dass eine Temperatur des zwischen der ersten Haltefläche (132) und der zweiten Haltefläche (172) gehaltenen lebenden Gewebes auf eine erste Temperatur (T1) erhöht wird, die ausreicht, um das lebende Gewebe durch die von der Energie erzeugte Wärme zu versiegeln, und das lebende Gewebe versiegelt wird; und
Zuführen der Energie zu dem Heizelement (126), so dass die Wärme des Heizelements (126) so konfiguriert ist, dass sie die erste Haltefläche (132) auf eine zweite Temperatur (T2) erwärmt, die höher als die erste Temperatur (T1) ist und ausreicht, um das lebende Gewebe, das zwischen der ersten Haltefläche (132) und der zweiten Haltefläche (172) gehalten wird, herauszuschneiden, und die vorstehende Fläche (136a) so konfiguriert ist, dass sie das lebende Gewebe herausschneidet, während sie die Temperatur des lebenden Gewebes von der ersten Temperatur (T1) auf die zweite Temperatur (T2) erhöht.

9. Behandlungssystem (10) nach Anspruch 7, wobei eine erste Temperatur (T1), die ausreicht, um das lebende Gewebe durch die von der Energie erzeugte Wärme zu versiegeln, in einem Bereich von 60 °C bis 200 °C eingestellt wird, und eine zweite Temperatur (T2), die höher als die erste Temperatur (T1) ist und ausreicht, um das lebende Gewebe zu entfernen, in dem Steuergerät (182) in einem Bereich von 220 °C bis 400 °C eingestellt wird.

10. Behandlungssystem (10) nach Anspruch 7, wobei das Steuergerät (182) so konfiguriert ist, dass es durchführt:
Erhöhen der Temperatur des lebenden Gewebes, das zwischen der ersten Haltefläche (132) und der zweiten Haltefläche (172) gehalten wird, auf eine erste Temperatur (T1) durch Wärme, die an dem lebenden Gewebe durch die Energie, die dem ersten Hochfrequenz-Elektrodenabschnitt (134) und dem zweiten Hochfrequenz-Elektrodenabschnitt (174) zugeführt wird, und die Energie des Heizelements (126) erzeugt wird, die so konfiguriert ist, dass sie die erste Haltefläche (132) erhitzt, so dass das lebende Gewebe versiegelt wird; und
Erhöhen der Energieabgabe an das Heizelement (126) im Vergleich zu einem Fall des Erhitzens des lebenden Gewebes auf die erste Temperatur (T1), um die erste Haltefläche (132) auf eine zweite Temperatur (T2) zu erhitzen, die höher ist als die erste Temperatur (T1) und ausreicht, um das zwischen der ersten Haltefläche (132) und der zweiten Haltefläche (172) gehaltene lebende Gewebe einzuschneiden, so dass das lebende Gewebe von der vorstehenden Fläche (136a) eingeschnitten wird.

11. Behandlungssystem (10) nach Anspruch 7, wobei das Steuergerät (182) so konfiguriert ist, dass es implementiert: Umschalten von einem Zustand, in dem die Energie dem ersten Hochfrequenz-Elektrodenabschnitt (134) und dem zweiten Hochfrequenz-Elektrodenabschnitt (174) zugeführt wird, um die Temperatur des lebenden Gewebes auf eine erste Temperatur (T1) zu erhöhen, die ausreicht, um das lebende Gewebe zu versiegeln, in einen Zustand, in dem die Energie des Heizelements (126) zugeführt wird, um die erste Haltefläche (132) auf eine zweite Temperatur (T2) zu erhitzen.

12. Behandlungssystem (10) nach Anspruch 7, wobei das Steuergerät (182) so konfiguriert ist, dass es implementiert: Zuführen der Energie zu dem Heizelement (126), um die erste Haltefläche (132) auf eine zweite Temperatur (T2) zu erwärmen, die höher ist als die erste Temperatur (T1) und ausreicht, um das lebende Gewebe zu exzidieren, während die Energie dem ersten Hochfrequenz-Elektrodenabschnitt (134) und dem zweiten Hochfrequenz-Elektrodenabschnitt (174) zugeführt wird, um die Temperatur des lebenden Gewebes auf eine erste Temperatur (T1) zu erhöhen, die ausreicht, um das lebende Gewebe zu versiegeln.

13. Behandlungssystem (10) nach Anspruch 7, wobei das Steuergerät (182) so konfiguriert ist, dass es eine konstante Temperaturregelung für das Heizelement (126) implementiert.

14. Behandlungssystem (10) nach Anspruch 7, wobei das Steuergerät (182) so konfiguriert ist, dass es eine Erhöhung der Temperatur des Heizelements (126) auf eine zweite Temperatur (T2), die ausreicht, um das lebende Gewebe schrittweise zu exzidieren, bewirkt.

## Revendications

1. Instrument de traitement (12) comprenant :
une première surface de retenue (132) qui comprend une première partie électrode haute fréquence (134) ;
une paire de secondes parties électrodes haute fréquence (174) qui comprend une seconde surface de support (172) en regard de la première partie de support (132), conçue pour retenir des tissus vivants en coopération avec la première surface de retenue (132), conçue pour alimenter en énergie haute fréquence les tissus vivants, et conçue pour augmenter une température des tissus vivants retenus entre la première partie électrode haute fréquence (134) et la seconde partie électrode haute fréquence (174) à une première température (T1) suffisante au moins pour dénaturer la protéine, pour sceller les tissus vivants par chaleur en coopération avec la première partie électrode haute fréquence (134) ;
une lame (136) qui est disposée sur la première surface de retenue (132), qui comprend une surface en saillie (136a) faisant saillie des premières surfaces de retenue (132), qui est formée au centre de la première surface de retenue (132) dans le sens de la largeur (β), et qui est conçue pour appliquer une pression sur les tissus vivants retenus entre la première et la seconde surfaces de retenue (132, 172) par la surface en saillie (136a) ; et
un élément chauffant (126) qui est conçu pour générer de la chaleur et pour alimenter en chaleur la première partie électrode haute fréquence (134), dans lequel l'élément chauffant est apte à chauffer la première surface de retenue (132) à une seconde température (T2) supérieure à la première température (T1) et suffisante pour inciser les tissus vivants au moyen de la lame (136), à la réception de l'énergie fournie au même moment ou après que l'énergie est fournie aux première et seconde parties électrode haute fréquence (134, 174) ;
dans lequel la seconde surface de retenue (172) comprend une partie de réception (166) ayant des propriétés d'isolation électrique, et est mise en contact avec la surface en saillie (136a) de la lame (136) ; et
dans lequel la partie de réception (166) a une forme concave, une surface plane ou lisse incurvée qui est disposée entre la paire de secondes parties électrode haute fréquence (174).

2. Instrument de traitement (12) selon la revendication 1, dans lequel la surface en saillie (136a) a des propriétés d'isolation électrique.

3. Instrument de traitement (12) selon la revendication 1, dans lequel la surface en saillie (136a) est formée d'un seul tenant avec la première surface de retenue (132).

4. Instrument de traitement (12) selon la revendication 1, dans lequel
la première surface de retenue (132) et la seconde surface de retenue (172) sont définies par un sens de la longueur (α) dans lequel la surface en saillie (136a) s'étend, un sens de la largeur (β) qui est orthogonal à la surface en saillie (136a) et un sens d'ouverture/fermeture (γ) dans lequel la première surface de retenue (132) et la seconde surface de retenue (172) sont ouvertes ou fermées, et
une distance entre la première surface de retenue (132) et la seconde surface de retenue (172) augmente à mesure que la première surface de retenue (132) et la seconde surface de retenue (172) se séparent de la surface en saillie (136a) dans le sens de la largeur (β).

5. Instrument de traitement (12) selon la revendication 1, dans lequel la première surface de retenue (132) est régulièrement continue vers la surface en saillie (136a).

6. Instrument de traitement (12) selon la revendication 1, dans lequel :
la première partie électrode haute fréquence (134) comprend une première surface inclinée qui s'incline à partir d'un centre vers une partie latérale de la première surface de retenue (132) dans le sens de la largeur (β),
la seconde partie électrode haute fréquence (174) comprend une seconde surface inclinée qui est en regard de la première surface inclinée et présente une inclinaison différente de celle de la première surface inclinée, et
lorsque les tissus vivants sont maintenus entre la première surface inclinée et la seconde surface inclinée, une distance entre la première surface inclinée et la seconde surface inclinée augmente à partir du centre vers la partie latérale de la première surface de retenue (132) dans le sens de la largeur (β).

7. Système de traitement (10) comprenant :
l'instrument de traitement (12) selon la revendication 1 ; et
un organe de commande (182) qui est conçu pour mettre en œuvre l'alimentation en énergie pour chacune parmi la première partie électrode haute fréquence (134), la seconde partie électrode haute fréquence (174) et l'élément chauffant (126) de l'instrument de traitement (12).

8. Système de traitement (10) selon la revendication 7, dans lequel l'organe de commande (182) est conçu pour mettre en œuvre :
l'alimentation en énergie de la première partie électrode haute fréquence (134) et de la seconde partie électrode haute fréquence (174) de sorte qu'une température des tissus vivants retenus entre la première surface de retenue (132) et la seconde surface de retenue (172) soit élevée à une première température (T1) suffisante pour sceller les tissus vivants au moyen de la chaleur générée par l'énergie et que les tissus vivants soient scellés ; et
l'alimentation en énergie de l'élément chauffant (126) de sorte que la chaleur de l'élément chauffant (126) soit adaptée pour chauffer la première surface de retenue (132) à une seconde température (T2) supérieure à la première température (T1) et suffisante pour exciser les tissus vivants retenus entre la première surface de retenue (132) et la seconde surface de retenue (172) et la surface en saillie (136a) est conçue pour exciser les tissus vivants tandis que la température des tissus vivants augmente de la première température (T1) à la seconde température (T2).

9. Système de traitement (10) selon la revendication 7, dans lequel une première température (T1) suffisante pour sceller les tissus vivants au moyen de la chaleur générée par l'énergie est définie dans une plage comprise entre 60 °C et 200 °C, et une seconde température (T2) supérieure à la première température (T1) et suffisante pour exciser les tissus vivants est définie dans une plage comprise entre 220 °C et 400 °C dans l'organe de commande (182).

10. Système de traitement (10) selon la revendication 7, l'organe de commande (182) étant conçu pour mettre en œuvre :
l'augmentation de la température des tissus vivants retenus entre la première surface de retenue (132) et la seconde surface de retenue (172) à une première température (T1) au moyen de la chaleur générée au niveau des tissus vivants par l'énergie fournie à la première partie électrode haute fréquence (134) et à la seconde partie électrode haute fréquence (174), et par l'énergie de l'élément chauffant (126) qui est conçue pour chauffer la première surface de retenue (132) de sorte que les tissus vivants soient scellés ; et
l'augmentation de l'énergie fournie à l'élément chauffant (126) en comparaison à un cas de chauffage des tissus vivants à la première température (T1) pour chauffer la première surface de retenue (132) à une seconde température (T2) supérieure à la première température (T1) et suffisante pour inciser les tissus vivants retenus entre la première surface de retenue (132) et la seconde surface de retenue (172) de sorte que les tissus vivants soient incisés par la surface en saillie (136a).

11. Système de traitement (10) selon la revendication 7, l'organe de commande (182) étant conçu pour mettre en œuvre : commuter d'un état dans lequel l'énergie est fournie à la première partie électrode haute fréquence (134) et à la seconde partie électrode haute fréquence (174) pour augmenter la température des tissus vivants, à une première température (T1) suffisante pour sceller les tissus vivants à un état dans lequel l'énergie est fournie à l'élément chauffant (126) pour chauffer la première surface de retenue (132) à une seconde température (T2).

12. Système de traitement (10) selon la revendication 7, l'organe de commande (182) étant conçu pour mettre en œuvre : l'alimentation en énergie de l'élément chauffant (126) pour chauffer la première surface de retenue (132) à une seconde température (T2) supérieure à la première température (T1) et suffisante pour exciser les tissus vivants tout en alimentant en énergie la première partie électrode haute fréquence (134) et la seconde partie électrode haute fréquence (174) pour augmenter la température des tissus vivants à une première température (T1) suffisante pour sceller les tissus vivants.

13. Système de traitement (10) selon la revendication 7, dans lequel l'organe de commande (182) est conçu pour mettre en œuvre le réglage de température constante pour l'élément chauffant (126).

14. Système de traitement (10) selon la revendication 7, dans lequel l'organe de commande (182) est conçu pour mettre en œuvre l'augmentation d'une température de l'élément chauffant (126) à une seconde température (T2) suffisante pour exciser les tissus vivants par étapes.
